# EUROPEAN PATENT SPECIFICATION

(11) **EP 2 933 636 B1**
(45) Date of publication and mention of the grant of the patent: **07.07.2021**
(21) Application number: 13863563.6
(22) Date of filing: 11.12.2013
(51) Int. Cl.: G01N 33/15, A01K 67/027, C12N 5/09, C12Q 1/24, G01N 33/50

(54) **PERSONALIZED ANTI-CANCER AGENT SCREENING SYSTEM**
SCREENING-SYSTEM FÜR PERSONALISIERTE KREBSMITTEL
SYSTÈME DE DÉPISTAGE D'AGENT ANTICANCÉREUX PERSONNALISÉ

(30) Priority: 11.12.2012 KR 20120143573
(43) Date of publication of application: 21.10.2015
(73) Proprietor: AIMED BIO INC., Songpa-gu Seoul (KR)
(72) Inventor: NAM, Do Hyun, Seoul 135-543 (KR); JOO, Kyeung Min, Seoul 158-055 (KR)
(74) Representative: Held, Stephan
(86) International application number: PCT/KR2013/011490
(87) International publication number: WO 2014/092457

(56) References cited:
- EP-A1- 1 792 978
- WO-A1-2005/014856
- WO-A2-2012/021899
- WO-A2-2012/145535
- KR-A- 20050 112 444
- KR-A- 20100 056 227
- US-A- 5 424 209
- US-A1- 2004 193 019
- US-A1- 2008 113 390
- US-A1- 2010 298 255
- US-A1- 2011 230 360
- SHIN-ICHI YAGUCHI ET AL: "Antitumor Activity of ZSTK474, a New Phosphatidylinositol 3-Kinase Inhibitor", JOURNAL OF THE NATIONAL CANCER INSTITUTE, OXFORD UNIVERSITY PRESS, GB, vol. 98, no. 8, 19 April 2006 (2006-04-19) , pages 545-556, XP008149295, ISSN: 0027-8874, DOI: 10.1093/JNCI/DJJ133

## Description

### TECHNICAL FIELD

The present invention relates to a method for screening personalized anticancer agents.

### BACKGROUND ART

As the human genome project has been performed, a base enabling the genetic understanding of human diseases was provided. Thus, it is expected that personalized medicine will be realized, in which various diseases are diagnosed, treated and prognosed for individual patients having different genetic environments.

In the prior art, a typical therapy for a specific disease (for example, hypertension) has been applied indiscriminately regardless of the kind or severity of disease. However, as medical technology has been developed, various therapies can now be applied to a specific disease. Thus, there is a need for the development of tailored medicine in which a therapy suitable for a patient can be selected depending on the patient's sex, age, physical constitution, occupation and regional environment. For example, when a patient afflicted with a specific disease visits a hospital, the patient consults with a doctor and undergoes a medical examination based on the condition, and the doctor presents a suitable therapeutic method on the basis of the examination results. When the doctor prescribes a drug for the patient, the drug is prescribed by the subjective decision of the doctor on the basis of the examination results. In some cases, the prescribed drug cannot show a desired therapeutic effect, and in this case, other drugs are prescribed. In order to minimize this trial and effort, there has been a need for the development of personalized medicine.

Generally, tailored medicine is also known as order-made medicine or personalized medicine. The term "tailored medicine" refers to a method that uses a suitable therapeutic method selected based on the results obtained by examining the physical constitution or environment of an individual patient. In order to realize genetic information-based tailored medicine, it is required to develop biomarkers for genetic diagnosis, various genetic examination methods, medical informatics analysis methods capable of analyzing/combining the genetic information obtained by the examination methods, targeted therapeutic techniques that can employ the analysis results obtained by the analysis methods, etc. In other words, it is required to develop not only technology capable of screening a therapeutic method suitable for a particular patient, but also technology capable of verifying the screened therapeutic method.

Although this tailored medicine can be applied to all diseases, it is expensive and time-consuming. For this reason, it will be effective to apply tailored medicine for the treatment of diseases that show severe symptoms while requiring long-term medical treatment, rather than applying tailored medicine to diseases such as simple traumas showing slight symptoms, or stomachaches having a short disease period.

Typical examples of diseases that show severe symptoms while requiring long-term medical treatment include cancer-related diseases. As known so far, patient's cancer cells show physiological characteristics that completely differ from those of normal cells, and such physiological characteristics are determined by the expression patterns of genes whose expression increases or decreases specifically in cancers compared to that in normal cells, and the expression patterns of such genes differ between patients or patient's tissues. Indeed, different patients having the same cancer are known to show different responses to the same anticancer agent, and this difference is believed to be attributable to the above-described characteristics of cancer diseases. For this reason, it is expected that the above-described tailored medicine will be effectively applied to cancer-related diseases, and thus studies thereon have been actively conducted.

For example, International Patent Application WO 2007/035842 discloses a method that comprises selecting suitable anticancer agents against patient's cancer and selecting one anticancer agent from the selected anticancer agents using the cancer cells of the patient. In addition, Korean Patent Laid-Open Publication No. 10-2012-0090850 discloses a method for selecting a cancer patient-tailored drug, which comprises measuring the expression levels of a plurality of target genes in a biological sample collected from a cancer patient, and selecting a drug that acts on a highly expressed target gene. However, these techniques related to tailored medicine have shortcomings in that, because the anticancer activities of various anticancer agents against a patient's sample should be measured and compared, the techniques are highly expensive and time-consuming, and in that a tailored anticancer agent can be selected only *in vitro,* and it is impossible to verify that the selected anticancer agent actually shows a desired effect in the patient.

Under such circumstances, the present inventors have made extensive efforts to develop a more improved method for selecting personalized therapeutic agents against cancer-related diseases, and as a result, have developed a combined system that includes both an *in vivo* screening method, which employs cancer cells collected from patients, an *in vivo* verification method, and have found that the use of the developed system makes it possible to more effectively screen and verify personalized therapeutic agents against cancer-related diseases, thereby completing the present invention.

US 2011/0230360 A1 describes a method for generating a personalized cancer drug treatment option report comprising obtaining a colon cancer sample from a subject; determining the status of one or more molecular markers in the sample; stratifying one or more cancer drug treatment options in the report based on the status of the one or more molecular markers; and annotating the report based on the status of the one or more molecular markers. An in vitro culture using said cancer cell sample and establishing a xenograft model can be effected in a further step.

EP 1792978 A1 describes a method for determining the effect of a test compound on a tumor stern cell, comprising the steps of: (a) contacting the tumor stern cell derived from a tumor with the test compound; (b) determining the response of the contacted cells to the test compound, or (c) trans-planting the tumor stern cell into an immunocompromised mouse; (d) administering a test compound to the immunocompromised mouse; and (e) determining the response of the transplanted tumor stem cell to the test compound; or (f) contacting the tumor stem cell with a test compound under conditions suitable to allow complex formation; and (g) detecting complex formation between the test compound and a tumor stem cell, wherein the presence of the complex identifies the test compound as specifically binding the tumor stem cell.

### DISCLOSURE OF INVENTION

### TECHNICAL PROBLEM

It is an object of the present invention to provide a system for screening personalized anticancer agents.

Another object of the present invention is to provide a method of screening personalized anticancer agents using the system for screening personalized anticancer agents.

Still another object of the present invention is to provide an apparatus for screening personalized anticancer agents.

### TECHNICAL SOLUTION

To achieve the above objects, the present invention provides a method of screening personalized anticancer agents using a system for screening personalized anticancer agents.

### ADVANTAGEOUS EFFECT

When a system for screening personalized anticancer agents is used, an anticancer agent showing an optimal anticancer activity against cancer cells collected from a patient can be selected from a variety of anticancer agents, and it is possible to previously examine a therapeutic response that can appear when the selected anticancer agent is administered into the patient. Thus, the risk of trial and error in cancer therapy can be reduced, and the cost and time required for cancer therapy can be reduced.

### BRIEF DESCRIPTION OF THE DRAWINGS

FIG. 1 is a schematic flowchart showing the technology of screening a personalized anticancer agent for a patient using the system for screening personalized anticancer agents.
FIG. 2 is a schematic flowchart of the technology of analyzing the correlation between the genetic information data of cancer, the clinical information data of a patient and personalized anticancer agent information data to construct an algorithm and selecting personalized anticancer agents using the constructed algorithm.
FIG. 3 is a schematic flowchart showing the technology of screening personalized anticancer agents using the apparatus for screening personalized anticancer agents.

### BEST MODE FOR CARRYING OUT THE INVENTION

The present invention provides a method for screening personalized anticancer agents, the method comprising the steps of:
(a) selecting candidate anticancer agents based on clinical and genetic information of a patient with cancer;
(b) treating cancer cells from a patient with candidate anticancer agents in vitro, measuring the anticancer activity of the treated candidate anticancer agents, and selecting a group of personalized anticancer agent candidates having anticancer activity in vitro, from the candidate anticancer agents; and
(c) treating a immunodeficient rodent model containing cancer cells from the patient with the selected candidate group of personalized anticancer agent candidates of step (b) to verify the anticancer effect of the candidate group in vivo,
   wherein steps (a) and (b) are performed with an avatar scanner apparatus,
   wherein the avatar scanner apparatus comprises a first means for selecting suitable candidate anticancer agents from the genetic information of the cancer, a second means for collecting cancer cells from a cancer tissue of the patient, and a third means for treating the cancer cells with the selected candidate anticancer agents and analyzing the effect of the candidate anticancer agents,
   wherein the avatar scanner apparatus includes a culture system, candidate anticancer agent reservoir, a candidate anticancer agent dispenser, a cell survival rate-measuring device, a data storage unit and a data analysis unit,
   wherein the culture system includes a medium reservoir, a cell dispenser, a medium exchanger, an incubator, a stirrer and a waste discharge unit.

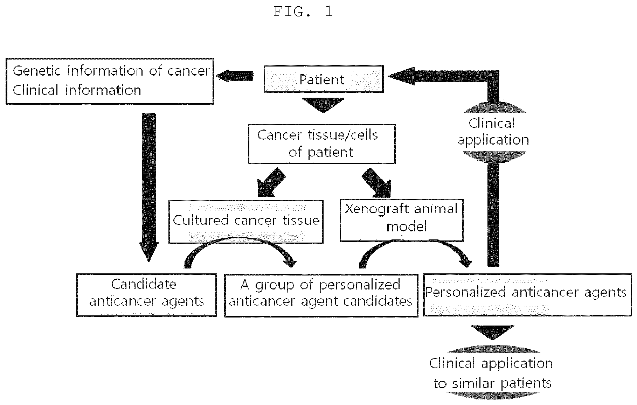

In the prior art, it was impossible to verify that an anticancer agent selected for a particular cancer patient in tailored medicine actually shows a proper effect in the patient. In order to overcome this shortcoming, the present inventors have developed a system comprising selecting candidate anticancer agents based on patient's information, treating cancer cells from the patient with the selected candidate anticancer agents, selecting a group of personalized anticancer agent candidates showing anticancer activity from the treated candidate anticancer agents, treating a xenograft animal model containing cancer cells from the patient with the candidate group, and selecting a personalized anticancer agent from the anticancer agents of the candidate group. The developed system was named "system for screening personalized anticancer drugs" or "avatar scan system" (see FIG. 1). FIG. 1 is a schematic flowchart showing a technology of screening a personalized anticancer agent for a patient using the system for screening personalized anticancer agents.

As shown in FIG. 1, candidate anticancer agents are selected based on the clinical information obtained from a patient and the genetic information of cancer, while cancer tissue or cells are collected from the patient. The collected cancer tissue or cells are cultured, and the cultured cancer tissue or cells are transplanted into an animal, thereby constructing a xenograft animal model. Then, the cultured cancer cells are treated with the selected candidate anticancer agents in order to select a group of personalized anticancer agent candidates from the anticancer agent candidates, and the xenograft animal model is treated with the group of personalized anticancer agent candidates in order to select a personalized anticancer agent from the candidate group. The selected personalized anticancer can be clinically applied to the patient to alleviate the symptoms of the patient or can also be clinically applied to similar patients having genetic symptoms similar to those of the patient to alleviate the symptoms of the similar patients.

Disclosed herewith is a system for screening personalized anticancer agents which may comprise the steps of: (a) selecting candidate anticancer agents based on information of a patient; (b) treating cancer cells from the patient with the selected candidate anticancer agents, and selecting a group of personalized anticancer agent candidates showing anticancer activity from the treated candidate anticancer agents; and (c) treating a xenograft animal model containing cancer cells from the patient with a candidate anticancer agent belonging to the determined candidate group in order to verify the effect of the anticancer agent. The present disclosure also provides a method for screening personalized anticancer agents, the method comprising the steps of: (1) treating cancer cells from a patient with candidate anticancer agents, and selecting a group of personalized anticancer agent candidates having anticancer activity from the candidate anticancer agents; and (2) treating a xenograft animal model containing cancer cells from the patient with the selected candidate group to verify the anticancer effect of the candidate group. The information of the patient in step (a) may be the genetic information of cancer or the clinical information of the patient, but is not limited thereto. When the information of the cancer is genetic information, including SNP obtained by analyzing the genotype of cancer cells, a haplotype, a mutation, a full-length nucleotide sequence, and the like, the genetic information may be applied to a drug response database to select candidate anticancer agents. In addition, even when only the information of the patient is used without using the genetic information of cancer, the candidate anticancer agents can be determined. Specifically, in this case, clinical information, including the site of cancer, the results of analysis of a cancer-specific gene marker, metastasis-related information, information about the symptoms of the patient, and the like, may be applied to a database including the clinical information of cancer and genetic information to select candidate anticancer agents.

Step (b) may be performed by treating cancer cells from the patient with the candidate anticancer agents, examining whether each of the treated candidate anticancer agents shows anticancer activity, and selecting a group of personalized anticancer agent candidates having anticancer activity from the candidate anticancer agents. In this step, the cancer cells from the patient may be, but are not limited to, either single cancer cells obtained by physically homogenizing cancer tissue from the cancer patient, centrifuging the homogenized tissue to collect a cell fraction, allowing the collected cell fraction to react with protease, and filtering and centrifuging the reaction product, or cultured cancer cells obtained by culturing the single cancer cells.

Step (c) may be performed using a xenograft animal model constructed by orthotopically transplanting cancer cells from the patient into an immunodeficient animal.

As used herein, the term "xenograft animal model" is also termed "avatar mouse" and refers to an animal model constructed by orthotopically transplanting cancer cells from the patient into an immunodeficient animal. The xenograft animal model is equal or similar to the cancer patient in terms of the morphological environment of cancer, the genetic environment, and the expression characteristics of marker proteins of cancer. Thus, the xenograft animal model can provide conditions reflecting the genetic, physiological and environmental characteristics of the cancer patients. Thus, when the xenograft animal model is treated with each of the personalized anticancer agents selected in step (b), it is possible to verify the same effect as that shown when the patient is treated with these anticancer agent candidates. Accordingly, the use of the xenograft animal model can overcome the shortcoming that it is impossible to verify that anticancer agents show a proper effect in patients. The present inventors previously constructed a xenograft animal model transplanted with glioblastoma (Korean Patent Application No. 10-2012-0067017, filed on June 21, 2012 and entitled "Glioblastoma xenograft animal model and use thereof").

As used herein, the term "immunodeficient animal" refers to an animal model constructed by artificially damaging a portion of the immune system to cause cancer and to make the immune system abnormal. The immunodeficient animal that is used in the present invention is an immunodeficient rodent, such as an immunodeficient mouse, rat, hamster or guinea pig. More preferably, it may be a nude mouse, a NOD (non-obese diabetic) mouse, a SCID (severe combined immunodeficiency) mouse, a NOD-SCID mouse, a NOG (NOD/SCID Il2rg-/-) mouse or the like, but is not limited thereto.

As used herein, the term "xenograft" means transplanting the liver, heart, kidney, organ, tissue or cell of an animal of a different species. For the purpose of the present invention, the term "xenograft" may mean transplanting cancer cells from a patient into an immunodeficient animal, but is not limited thereto.

In addition, in order to more easily perform the system for screening personalized anticancer agents, the system may further comprise, before step (b) of treating the cancer cells with the candidate anticancer agents, a step of cryopreserving a portion of the cancer cells. Herein, the cryopreservation may be performed using any method known in the art.

As used herein, the term "cancer" refers to solid cancer that comprises vessels and/or connective tissues and has a specific hardness and shape so as to able to be applied to the avatar scanner of the present invention. Preferably, the cancer may be liver cancer, glioblastoma, ovarian cancer, colorectal cancer, head and neck cancer, bladder cancer, hypernephroma, stomach cancer, breast cancer, metastatic cancer, pancreatic cancer, lung cancer or the like. More preferably, the cancer may be refractory cancer such as glioblastoma, metastatic brain cancer, triple-negative breast cancer, metastatic colorectal cancer, or pancreatic cancer, but is not limited thereto.

As used herein, the term "personalized anticancer agents" refers to pharmaceutical compositions that are determined using tailored medicine can show an optimal therapeutic effect specifically in a subject having cancer.

As used herein, the term "tailored medicine" is also termed "order-made medicine" or "personalized medicine" and a method that determines a suitable therapeutic method by examining the physical constitution or environment of an individual patient or treats the patient.

As used herein, the term "subject" means a living organism having or being at risk of developing cancer-related disease. Preferably, the subject may include mammals including humans, but is not limited thereto.

Meanwhile, the system for screening personalized anticancer agents may further comprise a step of applying the personalized cancer agent screening result for a preceding patient to the determination of a personalized anticancer agent candidate group for the next patient in order to increase the success rate of the screening of a personalized anticancer agent. In this case, the screened personalized anticancer agent data can be accumulated, and the screening efficiency can be increased using the accumulated data. For example, in the case in which a screening system for personalized anticancer agents that uses the genetic information of cancers from type A, type B and type C patients is constructed, when a personalized anticancer agent for a new C' type patient is screened using the above system, low screening efficiency is shown, but when the screening system for personalized anticancer agents is updated such that the C' type personalized anticancer agent-related information obtained by the above screening operation, the type C' personalized anticancer agent can be screened with high efficiency using the updated screening system. The operation of updating the system by adding data is preferably repeated.

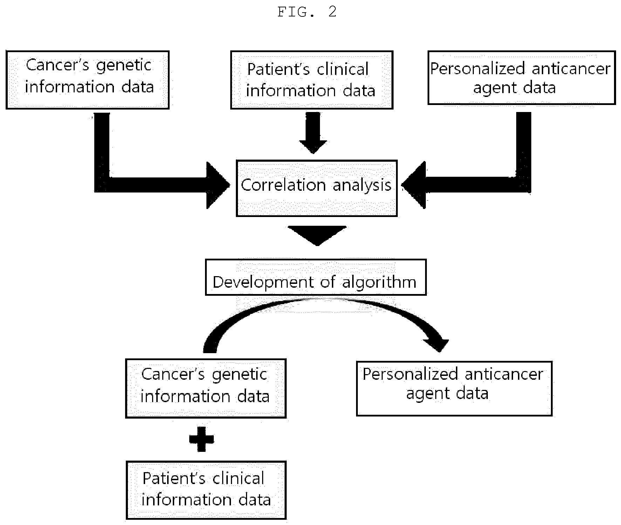

In another embodiment, when data are accumulated in the inventive screening system for personalized anticancer agents and the accumulated data are used, the correlation between the cancer's genetic information data used in the screening system, the patient's clinical information data and the personalized anticancer agent data obtained from the system can be analyzed, and an algorithm of predicting the correlation between these data can be developed using the analysis results. This algorithm makes it possible to link the data used in the personalized anticancer agent screening system to date selected from the system, and thus the use of the algorithm can construct a screening system so as to more easily select personalized anticancer drugs (see FIG. 2). FIG. 2 is a schematic flowchart of the inventive technology of analyzing the correlation between the genetic information data of cancer, the clinical information data of a patient and personalized anticancer agent information data to construct an algorithm and selecting personalized anticancer agents using the constructed algorithm. As shown in FIG. 2, when the correlation between the genetic information data of cancer, the clinical information data of a patient and personalized anticancer agent information data is analyzed, an algorithm capable of predicting the correlation between these data can be constructed. The constructed algorithm can be used to select personalized anticancer agents from the genetic information data of cancer and the clinical information data of patients without having to perform additional experiments *in vivo* and *in vitro.*

Preferably, the algorithm for selecting personalized anticancer agents from genetic information data and clinical information data may be constructed by the steps of: (a) selecting candidate anticancer agents from the genetic information data of cancer and the clinical information data of a patient; (b) treating cancer cells from the patient with the selected candidate anticancer agents, and selecting a group of personalized anticancer agent candidates showing anticancer activity from the treated candidate anticancer agents; (c) treating a xenograft animal model containing cancer cells from the patient with a candidate anticancer agent belonging to the determined candidate group in order to verify the effect of the anticancer agents, thereby screening personalized anticancer agents; (d) accumulating the genetic information data of cancer, the clinical information data of the patient, and the personalized anticancer agent data screened based thereon; and (e) analyzing the correlation between the accumulated genetic information data, clinical information data and personalized anticancer agent data. Herein, steps (a) to (c) are as described above. In addition, as described above, the algorithm may be updated by adding data, thereby increasing the success rate of screening of personalized anticancer agents.

Meanwhile, a system for screening personalized anticancer agents can be constructed using the above-described algorithm. For example, the system for screening personalized anticancer agents comprises the steps of: (a) accumulating the genetic information data of cancer, the clinical information data of a patient, and anticancer agent response information data; (b) analyzing the correlation between the accumulated data, thereby constructing an algorithm of selecting personalized anticancer agents from the data; and (c) selecting personalized anticancer agents from the genetic information data and the clinical information data of a patient using the constructed algorithm. Herein, step (a) is performed by the steps of: selecting candidate anticancer agents from the genetic information data of cancer and the clinical information data of a patient; treating cancer cells from the patient with the selected candidate anticancer agents, and selecting a group of personalized anticancer agent candidates showing anticancer activity from the treated candidate anticancer agents; treating a xenograft animal model containing cancer cells from the patient with a candidate anticancer agent belonging to the determined candidate group in order to verify the effect of the anticancer agents, thereby screening personalized anticancer agents; and accumulating the genetic information data of cancer, the clinical information data of the patient, and the personalized anticancer agent data screened based thereon. In addition, the method may further comprise, before step (c), a step of repeatedly performing a process of applying the results of screening of personalized anticancer agents for a preceding patient to determination of personalized anticancer agents for the next patient, thereby gradually increasing the success rate of personalized anticancer agents, and improving the constructed algorithm so as to reflect the repeated process.

In another aspect, the present disclosure provides an apparatus for screening personalized anticancer agents. The apparatus for screening personalized anticancer agents may comprise: (a) a first means for selecting suitable candidate anticancer agents from the genetic information of cancer; (b) a second means for collecting cancer cells from the cancer tissue of a patient; (c) a third means for treating the cancer cells with the selected candidate anticancer agents and analyzing the effect of the candidate anticancer agents. The system for screening personalized anticancer agents may also comprise: (1) a means for culturing cancer cells from a patient, comprising a medium reservoir, a cell dispenser, a medium exchanger, an incubator, a stirrer and a waste discharge unit; and (2) a means for treating cultured cancer cells from the patient with the candidate anticancer agents, measuring the anticancer activity of the treated candidate anticancer agents and selecting personalized anticancer agents showing an anticancer effect, the means comprising a candidate anticancer agent reservoir, a candidate anticancer agent dispenser, a cell survival rate-measuring device, a data storage unit and a data analysis unit.

Conventional technology related to tailored medicine for cancer patients has a shortcoming in that it is highly expensive and time-consuming. To overcome this shortcoming, the present inventors have developed an apparatus capable of effectively performing tailored medicine for cancer patients by rapidly and precisely performing steps of measuring and comparing the anticancer activity of various anticancer agents using a sample collected from an individual patient. This apparatus was termed "apparatus for screening personalized anticancer agents".

As used herein, the phrase "apparatus for screening personalized anticancer agents" is also termed "avatar scanner" and refers to an apparatus capable of screening personalized anticancer agents using cancer cells from a cancer patient. As described above, the apparatus comprises: a first means for selecting suitable candidate anticancer agents from the genetic information of cancer; a second means for collecting cancer cells from the cancer tissue of a patient; a third means for treating the cancer cells with the selected candidate anticancer agents and analyzing the effect of the candidate anticancer agents.

The first means may include a unit for storing data, including the information of a cancer patient and the information of candidate anticancer agents, and a driving algorithm for linking the information of the cancer patient with the information of candidate anticancer agents. The information of the cancer patient may include, but is not limited to, the genetic information of cancer, the site of cancer, the results of analysis of cancer-specific gene markers, metastasis-related information, the information of symptoms of the patient.

The second means may be a cancer cell extractor configured to perform a process of physically homogenizing cancer tissue from a cancer patient, centrifuging the homogenized material to collect a cell fraction, allowing the collected cell fraction to react with protease, and filtering and centrifuging the reaction product to obtain cancer cells. The cancer cell extractor may comprise a tissue homogenizer, a centrifuge, a protease reservoir, a cell filtration unit, a medium reservoir, and a waste discharge unit, but is not limited thereto.

The third means may be a HTS(high throughput screening) system configured to perform a process of treating the cancer cells, obtained by the second means, with one or more candidate anticancer agents selected by the first means, measuring the anticancer activity of the candidate anticancer agents and selecting personalized anticancer agents having anticancer activity. The HTS may comprise a candidate anticancer reservoir, a candidate anticancer agent dispenser, a peeler, a washer, a plate incubator, a cell survival rate measuring device, a data storage unit, and a data analysis unit. The data analysis unit may comprise a qPCR machine, a plate reader, a confocal microscope, a multiplex reader and the like.

Meanwhile, in order to more effectively use the apparatus for screening personalized anticancer agents, the apparatus may further comprise a cancer cell culture means configured to culture cancer cells by providing temperature, time and medium, which are suitable for the culture of cancer cells. Preferably, the cancer cell culture means may be a means for seed-culturing cancer cells or a means for expansion-culturing cancer cells. More preferably, the cancer cell culture means may be a cancer cell seed culture means or a cancer cell expansion means, which comprises a medium reservoir, a cell dispenser, a medium dispenser, a medium exchanger, an incubator, a stirrer, a waste discharge unit and the like, but is not limited thereto.

In addition, in order to more effectively use the apparatus for screening personalized anticancer agents, the apparatus may further comprise a cancer cryopreservation means configured to perform a process of treating the cultured cancer cells with a cryoprotective agent and cryopreserving the treated cancer cells. The cancer cryopreservation means may comprise a freezer, a quick freezer, a cryoprotective agent reservoir, a cryoprotective agent dispenser, a thawing device and the like, but is not limited thereto.

In addition, the apparatus for screening personalized anticancer agents may be configured to automatically perform extraction of cancer cells from cancer tissue collected from a cancer patient, seed culture of the extracted cancer cells, expansion culture of the seed-cultured cancer cells, cryopreservation of the cultured cells, treatment of the expansion-cultured cancer cells with candidate anticancer agents, and analysis of the effect of candidate anticancer agents.

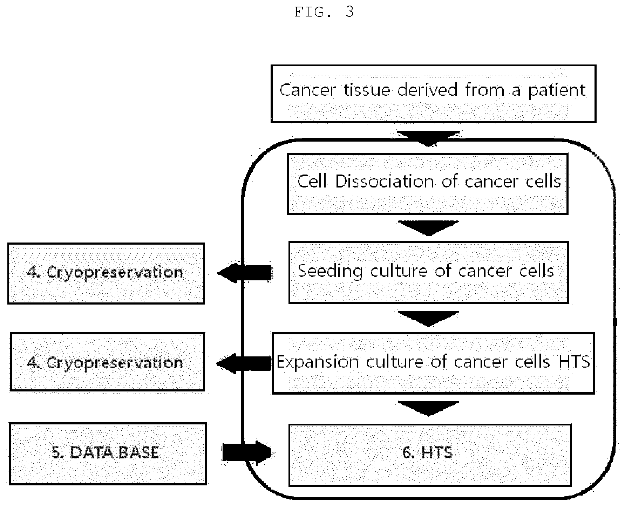

In an embodiment, the apparatus for screening personalized anticancer agents may comprise: a cancer cell extractor configured to obtain single cancer cells from cancer tissue collected from a cancer patient; a cancer cell seed-culture means configured to seed-culture the obtained single cancer cells; a cancer cell expansion-culture means configured to expansion-culture the seed-cultured cancer cells; a data storage unit including the information of the cancer patient and the information of candidate anticancer agents; a database comprising a driving algorithm that links the information of the cancer patient with the information of candidate anticancer agents; an HTS system configured to treat the expansion-cultured cancer cells with candidate anticancer agents selected from the database and analyze the effect of the candidate anticancer agents; and a cell cryopreservation unit (see FIG. 3). FIG. 3 is a schematic flowchart showing the technology of screening personalized anticancer agents using the apparatus for screening personalized anticancer agents. As can be seen in FIG. 3, extraction of cancer cells from cancer tissue derived from a patient (cell dissociation), seed culture of the extracted cancer cells, expansion culture of the seed-cultured cancer cells, and analysis (HTS) of effects of candidate anticancer agents, selected from a database, using the expansion-cultured cancer cells, are sequentially performed, and the cultured cancer cells may be cryopreserved.

## Claims

1. A method for screening personalized anticancer agents, the method comprising the steps of:
(a) selecting candidate anticancer agents based on clinical and genetic information of a patient with cancer;
(b) treating cancer cells from a patient with candidate anticancer agents in vitro, measuring the anticancer activity of the treated candidate anticancer agents, and selecting a group of personalized anticancer agent candidates having anticancer activity in vitro, from the candidate anticancer agents; and
(c) treating a immunodeficient rodent model containing cancer cells from the patient with the selected candidate group of personalized anticancer agent candidates of step (b) to verify the anticancer effect of the candidate group in vivo,
wherein steps (a) and (b) are performed with an avatar scanner apparatus,
wherein the avatar scanner apparatus comprises a first means for selecting suitable candidate anticancer agents from the genetic information of the cancer, a second means for collecting cancer cells from a cancer tissue of the patient, and a third means for treating the cancer cells with the selected candidate anticancer agents and analyzing the effect of the candidate anticancer agents,
wherein the avatar scanner apparatus includes a culture system, candidate anticancer agent reservoir, a candidate anticancer agent dispenser, a cell survival rate-measuring device, a data storage unit and a data analysis unit,
wherein the culture system includes a medium reservoir, a cell dispenser, a medium exchanger, an incubator, a stirrer and a waste discharge unit.

2. The method of claim 1, wherein the candidate anticancer agents in step (b) are screened based on the genetic information of cancer.

3. The method of claim 2, wherein the genetic information of cancer is at least one information selected from the results of analysis of a cancer-specific SNP, a cancer-specific haplotype, a cancerspecific mutation.

4. The method of claim 1, wherein the cancer cells of step (b) are obtained through the following steps:
(a) homogenizing cancer tissue isolated from the cancer patient, and collecting a cell fraction from the homogenized tissue; and
(b) allowing the collected cell fraction to react with protease, and filtering and centrifuging the reaction product.

5. The method of claim 1, wherein the cancer cells of step (b) are single cancer cells obtained from cancer tissue isolated from the cancer patient.

6. The method of claim 1, wherein the cancer cells of step (b) are obtained by culturing single cancer cells obtained from cancer tissue isolated from the cancer patient.

7. The method of claim 1, wherein the culture system includes a means for seed-culturing cancer cells or a means for expansion-culturing cancer cells.

8. The method of claim 1, wherein step (b) is performed by treating the cancer cells from the patient with the candidate anticancer agents, examining whether each of the treated candidate anticancer agents shows anticancer activity, and selecting a group of personalized anticancer agent candidates having anticancer activity from the candidate anticancer agents.

9. The method of claim 1, wherein the immunodeficient rodent is constructed by transplanting the cancer cells from the patient into a rodent.

10. The method of claim 9 1, wherein the immunodeficient rodent is an immunodeficient mouse.

11. The method of claim 10, wherein the immunodeficient mouse includes a nude mouse, a NOD (non-obese diabetic) mouse, a SCID (severe combined immunodeficiency) mouse, a NOD-SCID mouse, or a NOG (NOD/SCID 112rg-/-) mouse.

12. The method of claim 1, further comprising, before step step (b) of treating the cancer cells with the candidate anticancer agents, a step of cryopreserving a portion of the cancer cells.

13. The method of claim 1, wherein the cancer is solid cancer.

14. The method of claim 13, wherein the cancer is selected from the group consisting of liver cancer, glioblastoma, ovarian cancer, colorectal cancer, head and neck cancer, bladder cancer, hypernephroma, stomach cancer, breast cancer, metastatic cancer, pancreatic cancer, and lung cancer.

## Patentansprüche

1. Verfahren zum Screenen personalisierter Antikarzinommittel, wobei das Verfahren die folgenden Schritte umfasst:
(a) Auswählen in Frage kommender Antikarzinommittel auf der Basis klinischer und genetischer Informationen eines Patienten mit Karzinom;
(b) Behandeln von Karzinomzellen eines Patienten mit in Frage kommenden Antikarzinommitteln in vitro, Messen der Antikarzinomaktivität der angewandten in Frage kommenden Antikarzinommittel und Auswählen einer Gruppe personalisierter, in Frage kommender Antikarzinommittel, die in vitro eine Antikarzinomaktivität haben, aus den in Frage kommenden Antikarzinommitteln; und
(c) Behandeln eines Nagetiermodells mit Immundefekt, das Karzinomzellen des Patienten enthält, mit der ausgewählten in Frage kommenden Gruppe personalisierter, in Frage kommender Antikarzinommittel aus Schritt (b), um die Antikarzinomwirkung der in Frage kommenden Gruppe in vivo zu verifizieren,
wobei die Schritte (a) und (b) mit einer Avatar-Scanvorrichtung durchgeführt werden,
wobei die Avatar-Scanvorrichtung erste Mittel zum Auswählen geeigneter in Frage kommender Antikarzinommittel aus den genetischen Informationen des Karzinoms, zweite Mittel zum Sammeln von Karzinomzellen aus einem Karzinomgewebe des Patienten und dritte Mittel zum Behandeln der Karzinomzellen mit den ausgewählten in Frage kommenden Antikarzinommitteln und zum Analysieren der Wirkung der in Frage kommenden Antikarzinommittel umfasst,
wobei die Avatar-Scanvorrichtung ein Kultursystem, einen Behälter mit einem in Frage kommenden Antikarzinommittel, eine Ausgabevorrichtung für das in Frage kommende Antikarzinommittel, eine Vorrichtung zum Messen der Überlebensrate von Zellen, eine Datenspeichereinheit und eine Datenanalyseeinheit umfasst,
wobei das Kultursystem einen Mediumbehälter, ein Element zum Ausgeben von Zellen, ein Medium-Wechselelement, einen Inkubator, einen Rührer und eine Abfallabführeinheit umfasst.

2. Verfahren nach Anspruch 1, wobei die in Frage kommenden Antikarzinommittel in Schritt (b) auf der Basis der genetischen Informationen über das Karzinom gescreent werden.

3. Verfahren nach Anspruch 2, wobei die genetischen Informationen über das Karzinom Informationen sind, die aus den Ergebnissen einer Analyse eines karzinomspezifischen SNP, eines karzinomspezifischen Haplotyps und/oder einer karzinomspezifischen Mutation ausgewählt werden.

4. Verfahren nach Anspruch 1, wobei die Karzinomzellen von Schritt (b) durch die folgenden Schritte erhalten werden:
(a) Homogenisieren von Karzinomgewebe, das aus dem Karzinompatienten isoliert wurde, und Sammeln einer Zellfraktion aus dem homogenisierten Gewebe; und
(b) Ermöglichen, dass die gesammelte Zellfraktion mit Protease reagiert, und Filtern und Zentrifugieren des Reaktionsprodukts.

5. Verfahren nach Anspruch 1, wobei die Karzinomzellen von Schritt (b) einzelne Karzinomzellen sind, die aus Karzinomgewebe erhalten werden, das aus dem Karzinompatienten isoliert wurde.

6. Verfahren nach Anspruch 1, wobei die Karzinomzellen von Schritt (b) erhalten werden, indem einzelne Karzinomzellen kultiviert werden, die aus Karzinomgewebe erhalten werden, das aus dem Karzinompatienten isoliert wurde.

7. Verfahren nach Anspruch 1, wobei das Kultursystem Mittel für eine Seed-Kultivierung der Karzinomzellen oder Mittel für eine Expansion-Kultivierung der Karzinomzellen umfasst.

8. Verfahren nach Anspruch 1, wobei Schritt (b) durchgeführt wird, indem die Karzinomzellen des Patienten mit den in Frage kommenden Antikarzinommitteln behandelt werden, untersucht wird, ob jedes der angewandten in Frage kommenden Antikarzinommittel eine Antikarzinomaktivität zeigt, und eine Gruppe personalisierter, in Frage kommender Antikarzinommittel, die eine Antikarzinomaktivität haben, aus den in Frage kommenden Antikarzinommitteln ausgewählt wird.

9. Verfahren nach Anspruch 1, wobei das Nagetier mit Immundefekt durch Transplantieren der Karzinomzellen des Patienten in ein Nagetier konstruiert wird.

10. Verfahren nach Anspruch 1, wobei das Nagetier mit Immundefekt eine Maus mit Immundefekt ist.

11. Verfahren nach Anspruch 10, wobei die Maus mit Immundefekt eine Nacktmaus, eine NOD-Maus (nicht fettleibige diabetische Maus), eine SCID-Maus (Maus mit schwerem kombiniertem Immundefekt), eine NOD-SCID-Maus oder eine NOG-Maus (NOD/SCID Il2rg-/-) umfasst.

12. Verfahren nach Anspruch 1, das ferner vor Schritt (b) zum Behandeln der Karzinomzellen mit den in Frage kommenden Antikarzinommitteln einen Schritt der Kryokonservierung eines Teils der Karzinomzellen umfasst.

13. Verfahren nach Anspruch 1, wobei das Karzinom ein solides Karzinom ist.

14. Verfahren nach Anspruch 13, wobei das Karzinom aus der Gruppe ausgewählt wird, die aus Leberkarzinom, Glioblastom, Eierstockkarzinom, Kolorektalkarzinom, Kopf- und Halskarzinom, Blasenkarzinom, Nierenzellkarzinom, Magenkarzinom, Brustkarzinom, metastasierendem Karzinom, Pankreaskarzinom und Lungenkarzinom besteht.

## Revendications

1. Procédé de criblage d'agents anticancéreux personnalisés, le procédé comprenant les étapes suivantes :
(a) sélectionner des agents anticancéreux candidats sur la base d'informations cliniques et génétiques d'un patient atteint d'un cancer ;
(b) traiter des cellules cancéreuses d'un patient avec des agents anticancéreux candidats *in vitro,* mesurer l'activité anticancéreuse des agents anticancéreux candidats traités, et sélectionner un groupe de candidats agents anticancéreux personnalisés ayant une activité anticancéreuse *in vitro,* parmi les agents anticancéreux candidats ; et
(c) traiter un modèle de rongeur immunodéficient contenant des cellules cancéreuses du patient avec le groupe de candidats sélectionné de candidats agents anticancéreux personnalisés de l'étape (b) pour vérifier l'effet anticancéreux du groupe de candidats *in vivo,*
dans lequel les étapes (a) et (b) sont réalisées avec un appareil de balayage d'avatar,
dans lequel l'appareil de balayage d'avatar comprend un premier moyen de sélection d'agents anticancéreux candidats adaptés à partir des informations génétiques du cancer, un deuxième moyen de collecte de cellules cancéreuses d'un tissu cancéreux du patient, et un troisième moyen de traitement des cellules cancéreuses avec les agents anticancéreux candidats sélectionnés et l'analyse de l'effet des agents anticancéreux candidats,
dans lequel l'appareil de balayage d'avatar inclut un système de culture, un réservoir d'agents anticancéreux candidats, un distributeur d'agents anticancéreux candidats, un dispositif de mesure du taux de survie cellulaire, une unité de stockage de données et une unité d'analyse de données,
dans lequel le système de culture inclut un réservoir de milieu, un distributeur de cellules, un échangeur de milieu, un incubateur, un agitateur et une unité de décharge de déchets.

2. Procédé selon la revendication 1, dans lequel les agents anticancéreux candidats à l'étape (b) sont criblés sur la base des informations génétiques du cancer.

3. Procédé selon la revendication 2, dans lequel les informations génétiques du cancer sont au moins des informations sélectionnées parmi les résultats d'analyse d'un SNP spécifique du cancer, d'un haplotype spécifique du cancer, d'une mutation spécifique du cancer.

4. Procédé selon la revendication 1, dans lequel les cellules cancéreuses de l'étape (b) sont obtenues par les étapes suivantes :
(a) homogénéiser le tissu cancéreux isolé du patient cancéreux, et collecter une fraction cellulaire du tissu homogénéisé ; et
(b) laisser la fraction cellulaire collectée réagir avec la protéase, et filtrer et centrifuger le produit de réaction.

5. Procédé selon la revendication 1, dans lequel les cellules cancéreuses de l'étape (b) sont des cellules cancéreuses uniques obtenues d'un tissu cancéreux isolé du patient cancéreux.

6. Procédé selon la revendication 1, dans lequel les cellules cancéreuses de l'étape (b) sont obtenues par culture de cellules cancéreuses uniques obtenues d'un tissu cancéreux isolé du patient cancéreux.

7. Procédé selon la revendication 1, dans lequel le système de culture inclut un moyen de culture par ensemencement de cellules cancéreuses ou un moyen de culture par expansion de cellules cancéreuses.

8. Procédé selon la revendication 1, dans lequel l'étape (b) est réalisée par traitement des cellules cancéreuses du patient avec les agents anticancéreux candidats, par examen à la recherche d'une activité anticancéreuse présentée ou non par chacun des agents anticancéreux candidats traités, et par sélection d'un groupe de candidats agents anticancéreux personnalisés ayant une activité anticancéreuse parmi les agents anticancéreux candidats.

9. Procédé selon la revendication 1, dans lequel le rongeur immunodéficient est construit par transplantation des cellules cancéreuses du patient chez un rongeur.

10. Procédé selon la revendication 1, dans lequel le rongeur immunodéficient est une souris immunodéficiente.

11. Procédé selon la revendication 10, dans lequel la souris immunodéficiente inclut une souris nude, une souris NOD (diabétique non obèse), une souris SCID (déficit immunitaire combiné sévère), une souris NOD-SCID, ou une souris NOG (NOD/SCID Il2rg-/-).

12. Procédé selon la revendication 1, comprenant en outre avant l'étape (b) de traitement des cellules cancéreuses avec les agents anticancéreux candidats, une étape de cryoconservation d'une partie des cellules cancéreuses.

13. Procédé selon la revendication 1, dans lequel le cancer est un cancer solide.

14. Procédé selon la revendication 13, dans lequel le cancer est sélectionné dans le groupe consistant en un cancer du foie, un glioblastome, un cancer de l'ovaire un cancer colorectal, un cancer de la tête et du cou, un cancer de la vessie, un hypernéphrome, un cancer de l'estomac, un cancer du sein, un cancer métastatique, un cancer du pancréas, et un cancer du poumon.
